# EUROPEAN PATENT SPECIFICATION

(11) **EP 2 091 513 B1**
(45) Date of publication and mention of the grant of the patent: **06.04.2011**
(21) Application number: 07831968.8
(22) Date of filing: 09.11.2007
(51) Int. Cl.: A61K 9/00, A61K 47/18, A61K 38/00, A61K 47/20, A61M 15/00, B05B 1/02

(54) **EJECTION LIQUID AND EJECTION METHOD**
AUSSTOSSFLÜSSIGKEIT UND AUSSTOSSVERFAHREN
LIQUIDE D'ÉJECTION ET PROCÉDÉ D'ÉJECTION

(30) Priority: 04.12.2006 JP 2006327031
(43) Date of publication of application: 26.08.2009
(73) Proprietor: Canon Kabushiki Kaisha, Tokyo 146-8501 (JP)
(72) Inventor: MASADA, Yohei, Ohta-ku Tokyo 146-8501 (JP); SUGITA, Masaru, Ohta-ku Tokyo 146-8501 (JP); KANEKO, Hideki, Ohta-ku Tokyo 146-8501 (JP); SAKURADA, Naoko, Ohta-ku Tokyo 146-8501 (JP)
(74) Representative: TBK-Patent
(86) International application number: PCT/JP2007/072238
(87) International publication number: WO 2008/069012

(56) References cited:
- EP-A- 1 452 199
- WO-A-2006/035923
- WO-A-2006/035977
- WO-A-2006/107009
- WO-A-2006/118331

## Description

### TECHNICAL FIELD

The present invention relates to a liquid composition suitable for ejecting a liquid including at least one of a protein and a peptide, and an ejection method of the liquid composition, and an ejection device using the ejection method.

### BACKGROUND ART

Recently, a number of attempts to use as droplets protein solutions have been made. Examples of such attempts include applications of protein solution droplet formation techniques to transmucosal administration as a drug delivery method and to biochips and biosensors that need very small amounts of proteins. Additionally, in control of protein crystals and in screening of physiologically active substances, methods using protein microdroplets have attracted attention (see Japanese Patent Application Laid-Open No. 2002-355025, Allain, L. R. et al., "Fresenius J. Anal. Chem." 2001, Vol. 371, pp. 146-150, and Howard, E. I., Cachau, R. E., "Biotechniques" 2002, Vol. 33, pp. 1302-1306).

Additionally, proteins, in particular, enzymes and useful proteins having physiological activities are recently becoming mass producible owing to gene-recombination technology. Therefore, the process of making of protein into liquid droplets can be a useful technique for search, use and application fields of proteins as novel drugs. In particular, a technique to administrate biological substances including proteins, peptides and others as droplets from the lung has become significant. The lung has an alveolar surface area as large as 50 to 140 m², has the epithelium as an absorption barrier as very thin as 0.1 µm, and is additionally lower in enzymatic activity as compared to the digestive tract. Accordingly, transpulmonary administration has attracted attention as an administration route alternative to the injection of polymeric peptide drugs typified by insulin.

It has been generally known that the intrapulmonary deposition of the microdroplets of a drug is largely dependent on the pneumatic particle size of the microdroplets. Among others, for the delivery to the alveoli as the deep portion of the lung, droplets of 1 to 5 µm in particle size and narrow in particle size distribution are required to be administered with a high reproducibility.

As a method of preparing drug droplets with a narrow particle distribution, , an application of the principle of liquid ejection used in inkjet printing has been reported (see Japanese Patent Application Laid-Open No. 2002-248171). In the liquid ejection using the inkjet system, the liquid to be ejected is introduced into a small chamber, and a physical force is exerted on the liquid to eject droplets from an orifice. Examples of the ejection methods used include a method in which bubbles are generated by using electrothermal transducers such as thin film resistors to push out droplets from nozzles (thermal inkjet system), and a method in which the liquid is pushed out directly from orifices disposed on the top of the chamber by using electromechanical transducers such as piezooscillators (piezo inkjet system).

Generating droplets which composed of a protein or a peptide liquid, on the basis of the principle of the inkjet system, involves a problem such that the structure of the protein is destabilized due to the physical force (for example, pressure or shear force) exerted at the time of ejection or the high surface energy inherent to the microdroplets. When the thermal inkjet system is used, thermal energy is also applied to the drug solution. The steric structure of a protein or a peptide is vulnerable, and hence once the structure is broken, aggregation and decomposition of the protein or the peptide are caused, and the normal ejection is affected as the case may be. The above-described physical effect is extremely larger than the shear force or the thermal energy exerted by the usual stirring or heat treatment. (For example, in the thermal inkjet system, conceivably a load of 300°C and 90 atm is exerted instantly.) At the same time, two or more physical forces are applied. Consequently, the stability of the protein tends to be extremely easily degraded as compared to the conditions under which a protein is usually treated. When this problem is caused, the protein or the peptide is aggregated at the time of droplet preparation to cause clogging of a nozzle so as to make droplet ejection difficult.

Further, the droplets of 1 to 5 µm in particle size suitable for the pulmonary inhalation are smaller in particle size than the droplets for currently commercially available printers, and hence undergo larger surface energy and larger shear force. Consequently, it is extremely difficult to eject the protein and the peptide as microdroplets suitable for pulmonary inhalation.

Accordingly, it is essential to develop ejection liquids allowing proteins and peptides to be ejected stably.

On the other hand, known as a method for stabilizing a protein or a peptide is a method in which a surfactant, glycerol, various carbohydrates, a water-soluble polymer such as polyethylene glycol and albumin are added. However, frequently this method is hardly or not effective for improving the ejection stability in the ejection of a protein and a peptide by using the thermal inkjet system.

Additionally, even when a formulation is made to include additives suitable for inks used in inkjet printing, namely, moisturizing agents such as polyols including ethylene glycol and glycerin, and urea, such a formulation is hardly effective for improving the ejection performance in the ejection of a protein or a peptide.

As liquid compositions of proteins, peptides or the like suitable for generating droplets by using the thermal inkjet system, liquid compositions added with a surface tension regulating compound or a moisturizing agent are disclosed (see International Publication No. WO02/094342). In this case; the stability of a protein or a peptide in a droplets is claimed to be improved due to the surface tension, the viscosity and the moisturizing effect of the solution, and accordingly a surfactant and a water-soluble polymer such as polyethylene glycol are added. Liquid compositions added with a guanidine are also disclosed (see Japanese Patent Application Laid-Open No. 2006-117632).

However, in International Publication No. WO02/094342, no detailed description on the ejection stability is found. Further, according to the investigation of the present inventors, the addition of a surfactant or a water-soluble polymer has been found to exhibit insufficient effects when the concentration of the protein or the peptide is increased. Additionally, the present inventors have found many cases where no effect of the surfactant can be identified, and have found that the surface tension, the viscosity or the moisturizing effect does not regulate the ejection stability of the protein or peptide solution.

As described above, in the heretofore known protein solutions, the ejection stability in the thermal inkjet system has been found to be insufficient for some types of proteins and for some protein concentrations.

WO-A-2006/035977 is directed to an ejection liquid, wherein at least one compound having a guanidine group is added to an equal solution of proteins/peptides.

WO-A-2006/035923 is directed to an ejection liquid wherein amino acids, salts thereof and a surfactant is added to an equal solution containing proteins and/or peptides.

WO-A-2006/118331 and WO-A-2006/107009 are each being directed to ejection liquids containing proteins and/or peptides, wherein the stability of a protein/peptide in the liquid composition is improved by the addition of an amine or a compound having a betaine skeleton, respectively.

EP-A-1 452 199 is directed to a metered dose inhaler being a medicament ejector being suitable for ejecting a medicament.

### DISCLOSURE OF THE INVENTION

The present invention is based on the fact that a composition higher in ejection stability than conventional ejection liquids has been found. In other words, an object of the present invention is to provide an ejection liquid (liquid composition) for stable ejection of a solution including at least one of a protein and a peptide by taking advantage of thermal energy, and an ejection method suitable for ejecting the ejection liquid.

The ejection liquid of the present invention is defined according to claim 1.

The liquid ejection cartridge of the present invention is characterized by including a reservoir in which the above-described ejection liquid is stored, and an ejection head including an electrothermal transducer to impart thermal energy to the ejection liquid.

The liquid inhaler of the present invention is characterized by including the cartridge, and a suction port from which a user inhales the liquid ejected from the ejection head of the cartridge.

The ejection method of the present invention is

defined according to claim 7. According to the present invention, by adding a compound having in one molecule thereof a guanidine group, and a carboxyl group or a sulfonic acid group to a solution including al least one of a protein and a peptide, an ejection liquid allowing stable ejection based on an inkjet system is obtained. Generating liquid droplets by ejecting from a portable ejection device, and the user inhales the droplets, so at least one kind of a protein or a peptide which is medical properties can be transferred to the lungs and can be absorbed in the body. On the other hand, by ejecting the ejection liquid onto a substrate by using the above-described method, the ejection liquid can be used for preparing biochips and biosensors, for sensing, and for screening biological substances.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

FIG. 1 schematically illustrates an apparatus for forming a protein spot and a peptide spot on a substrate by using an ejection liquid according to the present invention.
FIG. 2 is a plan view illustrating an example in which protein spots and peptide spots are disposed on the surface of a substrate.
FIG. 3 is a schematic view illustrating a liquid ejection cartridge unit for use in an inhaler.
FIG. 4 is an oblique perspective view of an inhaler according to the present invention.
FIG. 5 is an oblique perspective view illustrating a condition in which an access cover is opened in FIG. 4.
FIG. 6 is a graph illustrating the ejection amounts of albumin solutions when ejected without adding the additives used in the present invention by using a thermal inkjet system.
FIG. 7 is a graph illustrating the ejection amounts of protein solutions when ejected with adding the additives used in the present invention by using a thermal inkjet system.
FIG. 8 is a graph illustrating the ejection amounts of protein solutions when ejected by using a thermal inkjet system with varied ejection frequencies.
FIG. 9 is a model view illustrating the experimental method of Example 132.

### BEST MODES FOR CARRYING OUT THE INVENTION

Preferred embodiments of the present invention will now be described in detail in accordance with the accompanying drawings.

The proteins as referred to in the present invention mean any polypeptides in which a large number of amino acids are bonded to each other through peptide bonds, capable of being dissolved or dispersed in aqueous solutions. Additionally, the peptides as referred to in the present invention mean the compounds in which two or more amino acids are bonded to each other through peptide bonds and the number of the amino acids is 50 or less. The proteins and the peptides may be chemically synthesized or prepared by purification of natural resources; typically, the proteins and the peptides are recombinants of natural proteins and peptides. By chemically modifying proteins and peptides through the covalent bonding of amino acid residues to protein molecules and peptide molecules, the effects of the proteins and the peptides can also be improved in such a way that the therapeutic effects of the proteins and the peptides are made to last longer.

In implementing the present invention, various proteins and peptides to be suitable for droplets can be used. Most typically, the droplets of proteins and peptides according to the present invention can be preferably used for the purpose of delivering therapeutically useful proteins and peptides to the lung.

Examples of the proteins and the peptides include: calcitonin; blood aggregation factors; ciclosporin; various hematopoietic factors such as G-CSF, GM-CSF, SCF, EPO, GM-MSF and CSF-1; interleukins such as IL-1 to 18; IGFs; M-CSF; thymosin; and cytokines inclusive of TNF and LIF. Further, examples of other usable proteins and peptides having therapeutic effects include: vasoactive peptides; interferons (alpha, beta, gamma or common interferon); growth factors or hormones such as human-growth hormones or other somatotropic hormones (such as bovine, swine or chicken growth factors); insulin; oxytocin; angiotensin; methionine enkephalin; substance P; ET-1; FGF; KGF; EGF; IGF; PDGF; LHRH; GHRH; FSH; DDAVP; PTH; vasopressin; glucagons; and somatostatin. Also used are protease inhibitors such as leupeptin, pepstatin and metallproteinase inhibitors (such as TIMP-1, TIMP-2 or other proteinase inhibitors). Also used are nerve growth factors such as BDNF and NT3. Also used are plasminogen activators such as tPA, urokinase and streptokinase. Also used are humanized various antibodies and receptors. Also used are peptide portions of proteins which portions have all or part of the main structures of the parent proteins and have at least part of the biological properties of the parent proteins. Also used are analogs such as substituted analogs or defective analogs, or modified amino acids such as peptide analogs, and substances containing the above-described substances modified with water-soluble polymers such as PEG and PVA. The fact that above-described proteins and peptides are able to be delivered to the lung is verified in Critical Reviews in Therapeutic Drug Carrier Systems, 12 (2&3) (1995).

Additionally, when the droplets of the present invention is applied to the preparation of biochips and biosensors and to the screening of proteins and peptides, various enzymes such as oxidase, reductase, transferase, hydrase, lyase, isomerase, synthetase, epimerase, mutase and lacease can be used in addition to the above-described proteins and peptides. There can also be used the above-described substances modified with reagents for immobilization by proteins and peptides to be used in diagnostic purposes such as various antibodies including IgG and IgE and receptors, and the corresponding antigens, and allergens, chaperonin, avidin and biotin.

As proteins and peptides to be contained in ejection liquids, proteins and peptides each having a molecular weight falling within a range, for example, from 0.5k to 150k Da can be used. Additionally, in an ejection liquid, the content of at least one selected from proteins and peptides is selected according to the purpose and application of the ejection liquid, and is selected preferably from a range of 1 µg/mL or more and 200 mg/mL or less, more preferably from a range of 0.1 mg/mL or more and 60 mg/mL or less.

It is to be noted that when a thermal inkjet system is applied to the present invention, the ejection performance exhibits the most remarkable improvement effect, and hence, the following description will be centered on a configuration based on the principle of the thermal inkjet system. However, in the present invention, the piezo inkjet system in which the liquid in the nozzle is ejected by using the vibratory pressure of a piezo element can also be used. The ejection system can be selected according to the type of the protein or the peptide to be ejected. In the present invention, among the inkjet systems, a mode in which thermal energy is imparted to the liquid by using an electrothermal transducer is expressed as the "thermal inkjet system," and a mode in which mechanical energy is imparted to the liquid by using an electromechanical transducer is expressed as the "piezo inkjet system", as is usually expressed in the field of printers. Although these terms are used for solutions of proteins, these terms each merely express that ejection energy is imparted to a solution on the basis of the principle of an "inkjet system".

For improvement of the ejection performance of the ink based on an inkjet system, addition of a surfactant or a solvent such as ethylene glycol is generally known. However, when a solution containing at least one of a protein and a peptide was ejected, no improvement of the ejection performance was found by merely adding these substances, and additives other than these substances were found to be necessary.

The present inventors have performed a diligent study, and consequently have found that protein solutions and peptide solutions each added with a compound having a guanidine group, and a carboxyl group or a sulfonic acid group in one molecule are suitable for stable ejection based on the inkjet system.

The reason why a compound having a guanidine group, and a carboxyl group or a sulfonic acid group in one molecule contributes largely to the ejection stability is not clear, but is conceivably as follows.

A guanidium group has a planar structure, and the amino group portion thereof works as a hydrogen donor so as to be able to form a hydrogen bond. The upper side and the under side of the molecular plane are covered with τ-electrons, and hence a guanidium group also has hydrophobicity. The peptide bonds in a protein and in a peptide are considered to be scarcely different in stability between when the peptide bonds form hydrogen bonds with guanidium groups and when the peptide bonds form hydrogen bonds with water or the peptide bonds mutually form hydrogen bonds. Further, a guanidium group has hydrophobic portions, and hence the hydrophobic portions of the guanidium group and the hydrophobic portions of the protein and the peptide interact with each other. By the fact that the amino group portion forms a hydrogen bond with water, the water solubilities of a protein and a peptide in the denatured state are increased, and hence the interaction of proteins and the interaction of peptides can be suppressed. Conceivably, the compound has an effect based on the suppression of the contact between proteins due to the negative charge carried by the concomitantly present carboxyl group or the concomitantly present sulfonic acid group. Conceivably, owing to these effects, the denaturation and the aggregation caused by the energy applied at the time of ejection based on the inkjet system are prevented, and hence the ejection is stabilized.

According to the present invention, the improvement effect of the ejection performance becomes remarkable, in particular, in a head to eject droplets by using thermal energy when the head is driven with a high frequency.

As the compound used in the present invention wherein the compound has a guanidine group, and a carboxyl group or a sulfonic acid group in one molecule, a compound represented by the following general formula (1) is preferable: 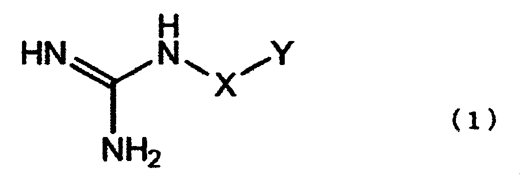 wherein in the above general formula (1), X represents an optionally branched alkyl group having one or more and eight or less carbon atoms. The number of the carbon atoms in the alkyl group X is not directly associated with the mechanism in which a protein is stably ejected owing to the compound; however, the number of carbon atoms is preferably one or more and eight or less, and more preferably one or more and four or less, for the purpose of maintaining the solubility of the molecule. One or more of the alkyl hydrogen atoms in X may be substituted with halogen atoms and hydroxy groups. When a side chain branched at X has a functional group or functional groups, the side chain has one or more hydroxyl groups, or carboxyl groups or sulfonic acid groups. Y represents a carboxyl group or a sulfonic acid group. Further, salts of these compounds may also be used. Additionally, polymers including any one of these compounds as one unit may also be used, and surfactants including any one of these compounds in the structure thereof may also be used.

Examples of the more preferable compounds among the compounds represented by the above-described general formula (1) include the compounds characterized in that in each of the compounds, X is an optionally branched alkyl group having one or more and eight or less carbon atoms, and when the alkyl group has a branched side chain, the branched side chain has a carboxyl group or a sulfonic acid group at the terminal of the side chain. Similarly, Y represents a carboxyl group or a sulfonic acid group. The salts of these compounds may also be used, a polymer including any one of these compounds as a unit may also be used, and a surfactant including any one of these compounds in the structure thereof may also be used.

The molecular weight of the compound having a guanidine group, and a carboxyl group or a sulfonic acid group in one molecule is preferably 117 or more and 2000 or less, and more preferably 117 or more and 500 or less. Additionally, for the purpose of obtaining the desired effects, it is preferable to use those compounds having a solubility in water of 0.1% by weight or more in a neutral region (pH 5.5 or more and 8.5 or less).

The preferable compounds are 2-guanidinoacetic acid, 3-guanidinopropionic acid, 4-guanidinobutanoic acid, α-guanidinoglutamic acid, guanidinomethanesulfonic acid, 2-guanidinoethanesulfonic acid, 3-guanidinopropanesulfonic acid, and the salts of these. The most preferable compounds are 3-guanidinopropionic acid and 2-guanidinoethanesulfonic acid, and the salts of these.

The compound is added in an amount of 0.1 part by weight or more and 50 parts by weight or less, more preferably 0.25 part by weight or more and 25 parts by weight or less, and most preferably 0.5 part by weight or more and 10 parts by weight or less, in relation to 1 part by weight of a protein or a peptide.

Although the addition concentration of the compound depends on the type and the concentration of the protein or the peptide, the addition concentration of the compound is preferably 0.001% by weight or more and 20% by weight or less, more preferably 0.05% by weight or more and 15% by weight or less, and most preferably 0.5% by weight or more and 10% by weight or less.

Further, in the present invention, it has also been found that the ejection stability can be maintained by adding the compound and a surfactant in combination even when the concentration of the additive is drastically decreased. The surfactant is added in an amount of 0.1 part by weight or more and 1 part by weight or less in relation to 1 part by weight of the compound. Accordingly, the addition amount of the compound to a solution with the same protein concentration can be reduced by a one-tenth from a half. In contrast to the compound, the effect of the surfactant conceivably includes stabilization of the ejection by suppressing the denaturation of the protein, and by dissolving the aggregated protein again. Conceivably, the combination of these two different effects leads to a synergetic effect to drastically improve the ejection stability. It is conceivable that with a surfactant alone, these effects are not large, so that the aggregation of a protein cannot be completely suppressed and hence the ejection stability cannot be ensured.

The surfactant as referred to in the present invention means a compound having both of a polar portion and a non-polar portion in one molecule, or a compound in which a polar portion and a non-polar portion are bonded to each other through a secondary bond such as an ionic bond. In other words, the surfactant has such characteristics that the surfactant reduces the interface tension through the molecular alignment thereof in the interface between two immiscible solvents, and is able to form micelles, wherein the polar portion and the non-polar portion are respectively located in localized regions separated away from each other in the molecule of the surfactant.

The usable surfactant is not particularly limited. However, typical examples of the surfactant include: sorbitan fatty acid esters such as sorbitan monocaprylate, sorbitan monolaurate and sorbitan monopalmitate; N-acyl amino acids each of which is a surfactant having an amino acid as the hydrophilic group thereof such as N-coconut oil fatty acid glycine, N-coconut oil fatty acid glutamic acid and N-lauroyl glutamic acid; fatty acid salts of amino acids; glycerin fatty acid esters such as glycerin monocaprylate, glycerin monomyristate and glycerin monostearate; polyglycerin fatty acid esters such as decaglyceryl monostearate, decaglyceryl distearate and decaglyceryl monolinolate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate; polyoxyethylene sorbitol fatty acid esters such as polyoxyethylene sorbitol tetrastearate and polyoxyethylene sorbitol tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether and polyoxyethylene polyoxypropylene cetyl ether; polyoxyethylene alkylphenyl ethers such as polyoxyethylene nonylphenyl ether; polyoxyethylene hardened castor oils such as polyoxyethylene castor oil and polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; surfactants having HLB 6 to 18 such as polyoxyethylene fatty acid amides including polyoxyethylene stearic acid amide; anionic surfactants such as alkyl sulfates each having an alkyl group having 8 to 18 carbon atoms including sodium cetyl sulfate, sodium lauryl sulfate and sodium oleyl sulfate; polyoxyethylene alkyl ether sulfates each having an average addition number of moles of 2 to 4 of ethylene oxide and 8 to 18 carbon atoms in the alkyl group such as sodium polyoxyethylene lauryl sulfate; alkyl benzene sulfonates each having 8 to 18 carbon atoms in the alkyl group such as sodium lauryl benzene sulfonate; alkyl sulfosuccinates each having 8 to 18 carbon atoms in the alkyl group such as sodium lauryl sulfosuccinate; natural surfactants such as lecithin and glycerophospholipid; sphingophospholipid such as sphingomyelin; and saccharose fatty acid esters of fatty acids each having 8 to 18 carbon atoms. To the ejection liquid (liquid composition) of the present invention, these surfactants can be added each alone or in combinations of two or more thereof.

Preferable surfactants are polyoxyethylene sorbitan fatty acid esters; particularly preferable among these are polyoxyethylene 20 sorbitan monolaurate, polyoxyethylene 20 sorbitan monostearate, polyoxyethylene 20 sorbitan tristearate, polyoxyethylene 20 sorbitan monolaurate, N-coconut oil fatty acid glycine, N-coconut oil fatty acid glutamic acid, N-lauroyl glycine, N-lauroyl glutamic acid, N-lauroyl sarcosine, lauramide propyl betaine, coconut oil fatty acid salt of arginine; and most preferable are polyoxyethylene 20 sorbitan monolaurate, polyoxyethylene 20 sorbitan monooleate, N-coconut oil fatty acid glycine, N-coconut oil fatty acid glutamic acid, N-lauroyl sarcosine, lauramide propyl betaine and coconut oil fatty acid salt of arginine. Additionally, particularly preferable for pulmonary absorption are polyoxyethylene 20 sorbitan monolaurate and polyoxyethylene 20 sorbitan monooleate.

Although the addition concentration of the surfactant depends on the type and content of the concomitantly present protein or peptide, the addition concentration of the surfactant falls preferably in a range of 1 µg/mL or more and 1.0 g/mL or less, more preferably in a range of 0.1 mg/mL or more and 100 mg/mL or less, and most preferably in a range of 0.2 mg/mL or more and 50 mg/mL or less, and is selected from the concentrations of the critical micelle concentration or more.

The composition of the liquid medium is preferably, from the viewpoint of the solubility of the protein or the like, such that water is predominant and the water proportion in the medium is 50% or more. There can be used mixed liquid media containing water-soluble organic solvents such as alcohols in addition to the main component of the medium, namely, water.

Specific examples of the water-soluble organic solvent include: amides such as dimethylformamide and dimethylacetamide; ketones such as acetone; ethers such as tetrahydrofuran and dioxane; ethanol; polyalkylene glycols such as polyethylene glycol and polypropylene glycol; alkylene glycols with an alkylene group having 2 to 6 carbon atoms such as ethylene glycol, propylene glycol, butylene glycol, triethylene glycol, 1,2,6-hexane triol, thiodiglycol, hexylene glycol and diethylene glycol; glycerin; lower alkyl ethers of polyalcohols such as ethylene glycol monomethyl (or ethyl) ether, diethylene glycol monomethyl (or ethyl) ether, and triethylene glycol monomethyl (or ethyl) ether; and N-methyl-2-pyrrolidone.

In the embodiments of the present invention, for the purpose of eliminating microbial effects, an antimicrobial agent, a germicidal agent, or an antiseptic agent may be added. Examples of those agents include: quaternary ammonium salts such as benzalkonium chloride and benzatonium chloride; phenol derivatives such as phenol, cresol and anisole; benzoic acids such as benzoic acid and paraoxybenzoic acid ester; and sorbic acid.

In the embodiments of the present invention, for the purpose of increasing the physical stability of an ejection liquid at the time of storage, an oil, glycerin, ethanol, urea, cellulose, polyethylene glycol, alginic acid salts and nicotinamide may be added. Additionally, for the purpose of increasing the chemical stability, ascorbic acid, citric acid, cyclodextrin, tocopherol or any other antioxidants may be added.

For the purpose of regulating the pH of the ejection liquid, a buffer may be added to the ejection liquid. For example, the following buffers may be used: ascorbic acid, citric acid, diluted hydrochloric acid, and diluted sodium hydroxide; and additionally, sodium hydrogen phosphate, sodium dihydrogen phosphate, potassium hydrogen phosphate, potassium dihydrogen phosphate, PBS, HEPES and Tris buffer.

As an isotonizing agent, aminoethylsulfonic acid, potassium chloride, sodium chloride, glycerin or sodium hydrogen carbonate may be added.

Additives used for inkjet ink such as ethylene glycols, urea, xylitol, EDTA and taurine may also be added.

When the ejection liquid according to the present invention is used as a spray liquid, saccharides such as glucose and sorbitol, sweetening agents such as aspartame, menthol and various flavoring ingredients may be added as scenting and flavoring agents. Further, hydrophobic compounds and oily compounds as well as hydrophilic compounds may also be used.

Further, according to need, various additives, suitable for intended applications of the ejection liquid, such as a surface modifier, a viscosity modifier, a solvent and a moisturizing agent can be added in appropriate amounts. Specific examples of such mixable additives include: a hydrophilic binder, a hydrophobic binder, a hydrophilic thickener, a hydrophobic thickener, glycol derivatives, alcohols and electrolytes; these may be added each alone or in combinations of two or more thereof. It is to be noted that it is more preferable to use, as various substances to be used as the above-described additives, the additives which are for medicinal use and are listed in pharmacopoeias of various countries as subsidiary components allowed to be added, or the additives allowed to be used in food and cosmetics, in preparing therapeutic liquid formulations.

The addition proportion of each of the various substances to be added as the above-described additives is varied depending on the types of the target proteins and peptides; however, in general, the addition proportion of each of such substances is selected so as to fall preferably in a range of 0.001% or more and 40% or less by weight, and more preferably 0.01% or more and 20% or less by weight. The addition amount of each of the above-described additives is varied depending on the type, quantity and combination, but is preferably 0.1 part by weight or more and 200 parts by weight or less in relation to 1 part by weight of the protein and peptide, from the viewpoint of the ejection performance.

Additionally, when a protein solution and a peptide solution are ejected by using the thermal inkjet system, the driving frequency of the head is preferably as lower as possible. The "driving frequency" as referred to in the present invention means the number of the ejection pulses per second applied to an electrothermal transducer in the case of the thermal inkjet system. The reason why the ejection stability is varied depending on the driving frequency is conceivably that when the ejection liquid is heated with the electrothermal transducer of the thermal inkjet head, the protein and the peptide become partially insoluble in water, and consequently the transfer of the energy from the electrothermal transducer to the solution is hindered. When the driving frequency is low, the temporarily formed insoluble substances, if any, is dissolved again by the subsequent driving time; on the other hand, when the driving frequency is high, the recovery of the dissolution becomes insufficient, and conceivably the ejection stability is thereby degraded. However, for the purpose of efficiently ejecting a large amount of a solution, ejection is required to be carried out with frequencies of a certain value or higher. In the present invention, the driving frequency is preferably 0.1 kHz or higher and 100 kHz or lower, and more preferably 1 kHz or higher and 30 kHz or lower.

When the ejection liquid according to the present invention is used in preparation of biochips and biosensors and in screening of proteins and peptides, a system almost the same as the systems in currently commercially available inkjet printers can be used.

On the other hand, the liquid inhaler according to the present invention includes a thermal inkjet head capable of ejecting microdroplets of an ejection liquid on the basis of the thermal inkjet system. It is preferable to adopt a mechanism capable of independently driving the individual electrothermal transducers of the nozzles constituting the head. In this case, the electrical connectors for use in connecting a plurality of control signals required for the independent driving of the individual electrothermal transducers and the wirings connecting the individual electrothermal transducers are integrated. Additionally, it is preferable to adopt a form of a liquid ejection cartridge in which the reservoir storing the ejection liquid and the ejection head including the electrothermal transducers imparting thermal energy to the ejection liquid are integrated.

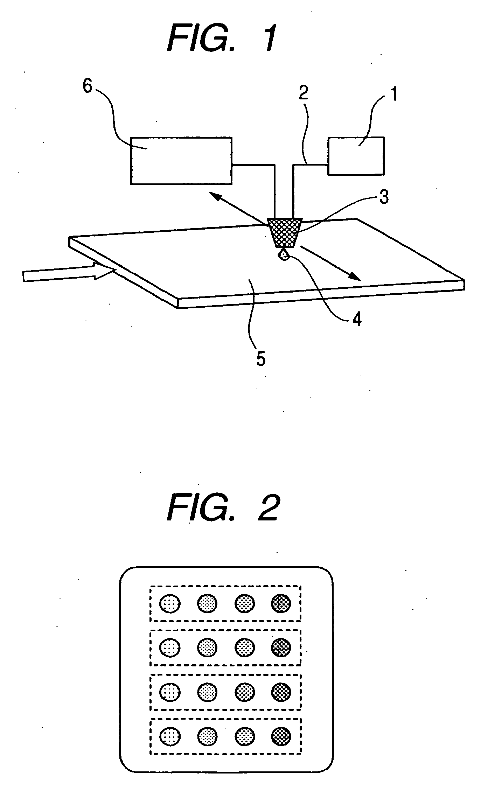

FIG. 1 schematically illustrates an apparatus for forming protein spots and peptide spots on a substrate by using the ejection liquid according to the present invention. The substrate 5 is used as a detection plate on which formed are immobilization areas of reference substances such as proteins, peptides, enzymes and antibodies for use in detecting various substances contained in a sample. An ejection head 3 includes at least a liquid path (not shown) for imparting ejection energy to the liquid and an ejection nozzle (not shown) communicatively connected to the liquid path. Ejection energy is imparted to the liquid fed through a liquid feed path 2 to the liquid path from a reservoir 1 storing the liquid, and consequently the liquid is ejected as a droplet 4 from an ejection nozzle on a predetermined position on the surface of the substrate 5. The ejection head 3 is disposed on a carriage capable of positioning along the plane direction shown by the arrows, and the landing position of the droplet 4 on the substrate 5 is adjusted by moving the ejection head 3. The ejection timing of the droplet 4 is controlled by a controller 6 electrically connected to the ejection head 3.

FIG. 2 illustrates a plan view of an example in which protein spots and peptide spots are disposed on the surface of a substrate. In the figure, the color density differences of the spots represent the differences in the protein and peptide concentrations, wherein each of the areas encircled with dotted lines is prepared by spotting with one ejection liquid. In the example shown, a plurality of ejection units capable of respectively ejecting different ejection liquids and capable of being driven independently can be disposed in the ejection head portion. By connecting the feeding systems of predetermined ejection liquids to the individual units respectively, two or more types of spots can be formed on the substrate. Further, by varying the liquid amounts imparted to the respective spot formation positions, spots with different attached amounts can be formed.

Next, description is made on a case where the ejection liquid according to the present invention is used for spraying, in particular, a case where the concerned ejection liquid is applied to an inhaler. It is preferable to use an inhaler having a structure in which a part for converting the ejection liquid into microdroplets and a part for mixing the sprayed microdroplets in a conveying gas flow are included independently. In this way, by separating the part for converting into microdroplets and the part for forming the gas flow containing the microdroplets, the ejection amount can be controlled to be more uniform. In other words, when the gas flow is inhaled into a person to be administered, the amount of a protein or a peptide as an effective component, namely, the predetermined amount of the component in the gas flow in every single administration can be adjusted more uniformly. As described above, by making the ejection head portion have a structure in which the plurality of ejection units each having a large number of ejection nozzles eject respectively different effective components, the ejection amounts of two or more effective components can also be controlled.

Downsizing of an inhaler portable by a user is facilitated by using, as the ejection head serving as a spray mechanism, an ejection head based on the principle of the thermal inkjet system in which ejection nozzles can be disposed with a high density per unit area.

In an inhaler for pulmonary inhalation, it is important that the particle size of the droplets contained in a gas flow is 1 to 5 µm, and exhibits a narrow particle size distribution. Further, when such an inhaler is used as a portable inhaler, the inhaler is required to have a compact structure.

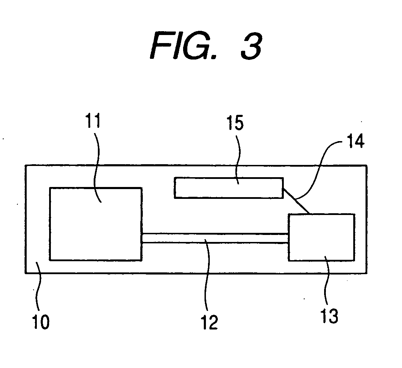

FIG. 3 schematically illustrates an example of a liquid ejection cartridge in such an inhaler. The liquid ejection cartridge has a structure as a head cartridge unit in which integrated in a housing 10 are a head 13, a reservoir 11 for storing an ejection liquid, a liquid path 12 for feeding the liquid from the reservoir 11 to the head 13, an electrical connector 15 for exchanging the driving signals and the controlling signals with a controller for controlling the driving of the individual ejection units of the head 13, and an internal wiring 14 between the head 13 and the electrical connector 15. The head cartridge unit is designed to have a structure capable of being detachable from the inhaler according to need. As the head 13, preferable is a head having the structure of a droplet ejection head described in Japanese Patent Application Laid-Open No. 2003-154665.

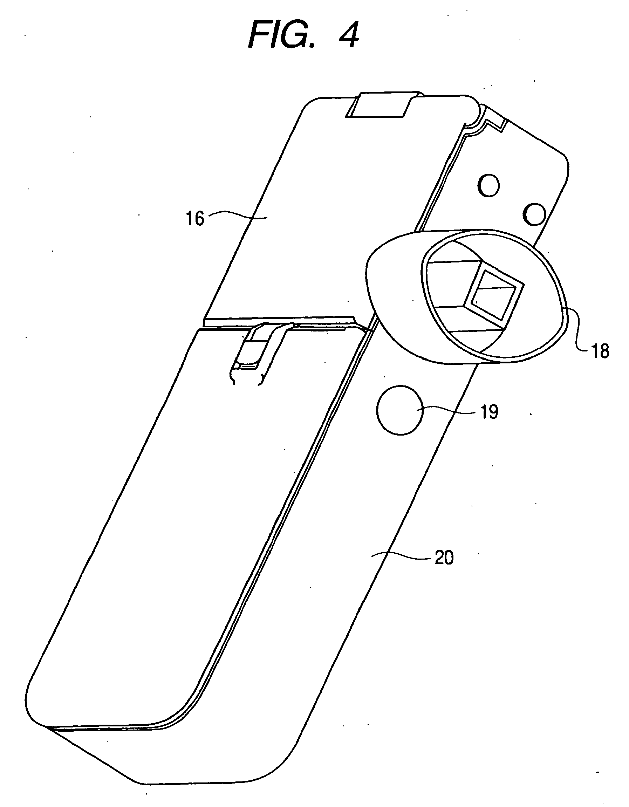

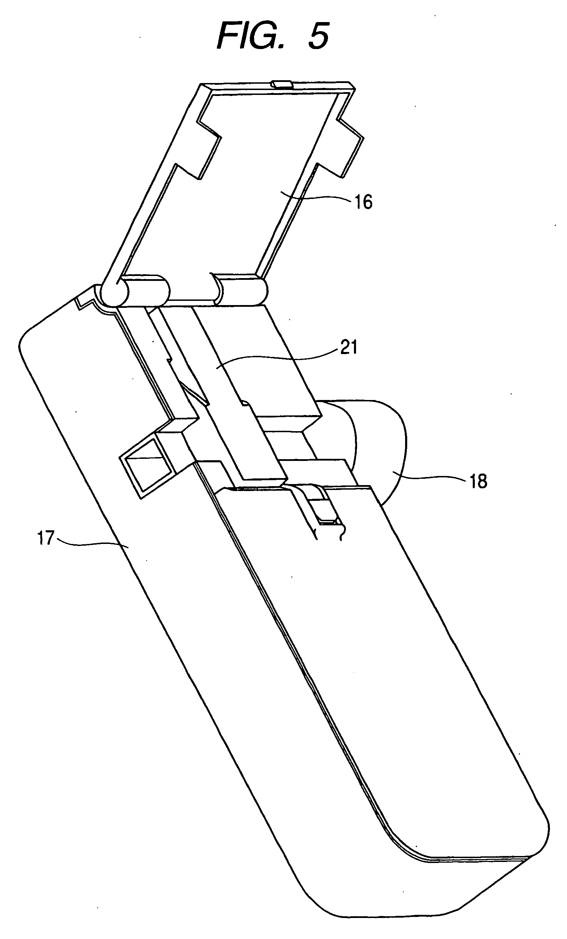

With reference to FIGS. 4 and 5, description is made on an example of a portable inhaler including a head cartridge unit having such a structure as described above. The inhaler illustrated in FIGS. 4 and 5 shows the structure of an example downsized so as to be portable by a user for a therapeutic purpose.

FIG. 4 is an oblique perspective view showing the appearance of an inhaler. In the inhaler, the housing of the inhaler is formed of the inhaler main body 20 and an access cover 16. The housing further houses a controller, a power source (battery) and the like (not shown). FIG. 5 is an oblique perspective view illustrating a condition in which an access cover 16 is opened, and when the access cover 16 is opened, a head cartridge unit 21 can be seen. By the inhale operation by a user, air is inhaled into the inhaler from an air intake opening 17 and is guided into a mouthpiece 18 (suction port) so as to stay there; the air is mixed with droplets ejected from the ejection nozzles formed in the head 13 of the head cartridge unit 21 so as to prepare a mixed gas flow. The mixed gas flow heads over to the mouthpiece exit having a shape suitable for being taken in mouth. When a user inserts into the mouth the tip of the mouthpiece and holds the mouthpiece with the teeth, and takes in breath, the droplets ejected from the liquid ejection portion are guided into the mouthpiece 18 and can be inhaled effectively.

It is to be noted that the head cartridge unit 21 can be made to have a structure capable of being detachable from the inhaler according to need.

By adopting the structure illustrated in FIGS. 4 and 5, the formed microdroplets are made to be able to spontaneously reach the throat and the interior of the trachea of a person to be administered along with the inhaled air. Accordingly, the amount of the sprayed liquid (the administration amount of the effective components) can be controlled independently of the magnitude of the inhaled air volume.

### (Referential Example 1)

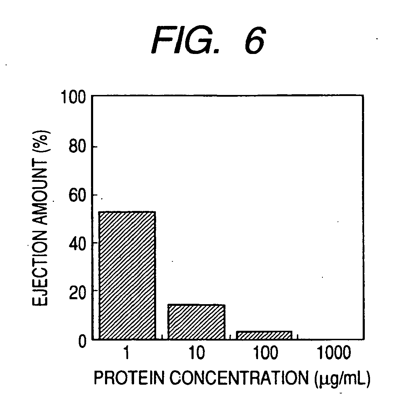

Before starting the description on Examples, for the purpose of further understanding the difficulty in ejection of protein solutions, the ejection amounts obtained in the cases where merely a protein was ejected with the thermal inkjet system are shown. The protein solutions in which bovine serum albumin (BSA) was dissolved in a phosphate buffer solution (PBS) were used. The solutions with various concentrations were ejected by using a liquid ejection apparatus fabricated by modifying a Bubblejet (trade mark) printer (trade name: PIXUS950i; manufactured by Canon Inc.) so as to be able to recover the solutions. The ejection amount (the amount of a droplet) of pure water obtained by ejecting in the same manner as in the ejection of the protein solutions was assumed to be 100%, and on the basis of this assumption, the ejection amount (the amount of a droplet) of each of the individual albumin solutions was presented. The results thus obtained are shown in FIG. 6.

Even with a BSA concentration as low as 1 pg/mL, the ejection stability was not perfect, and with further increasing protein concentration, the ejection amount was varied so as to gradually diminish. If the ejection amount largely varies depending on the protein concentration, for example, when the spots of a protein and a peptide are quantitatively disposed on a substrate, it is difficult to adjust so as to obtain the desired protein and peptide concentrations. Further, also when an ejection apparatus is used as an inhaler, the adjustment of the protein and peptide concentrations of an ejection liquid becomes difficult in uniformalizing the protein and peptide amounts in every single administration. Further, in an inhaler, ejection is required to be carried out with a further smaller droplet size, and hence the ejection of the protein and peptide solutions conceivably becomes difficult.

Hereinafter, the present invention is described in more detail with reference to Examples, but the present invention is not limited to these Examples. It is to be noted that "%" means "% by weight."

The compounds, used in Examples, each having a guanidine group, and a carboxyl group or a sulfonic acid group in one molecule, namely, 3-guanidinopropionic acid and 4-guanidinobutanoic acid were purchased from Sigma Aldrich, Inc. Additionally, 2-guanidinoacetic acid, α-guanidinoglutamic acid, guanidinomethanesulfonic acid, 2-guanidinoethanesulfonic acid and 3-guanidinopropanesulfonic acid were synthesized with reference to Tetrahedron Letters, Vol. 33, pp. 5933-5936 (1992), Tetrahedron, Vol. 57, pp. 7073-7105 (2001), and Japanese Patent Application Laid-Open No. 2002-502378.

### (Examples 1 to 14 and Comparative Examples 1 to 14)

### (Ejection of Protein Solutions Based on the Principle of the Thermal Inkjet System)

The preparation procedure of an ejection liquid is shown below. In ultrapure water, a protein was beforehand dissolved so as to have an appropriate concentration. A compound having a guanidine group, and a carboxyl group or a sulfonic acid group in one molecule was added to the thus prepared solution under stirring. The solution was adjusted to have a pH 7.0 with a 0.1 M NaOH or a 0.1 M HCl, and then the solution was adjusted in volume by adding ultrapure water so as to have a predetermined insulin concentration.

The ejection liquid prepared according to the above-described procedure was filled in a head of a Bubblejet (trade mark) printer (trade name: PIXUS950i; manufactured by Canon Inc.), and by driving the ejection head with an ejection controller, ejection was carried out at a frequency of 500 Hz. From the time used for the ejection; the number of ejection shots was calculated to be compared among the liquids.

Before and after the ejection, each ejection liquid was subjected to a HPLC analysis (the measurement conditions: apparatus: JASCO Corp., column: YMC-Pack Diol-200, 500 x 8.0 mm ID; eluent: 0.1 M KH₂PO₄-K₂HPO₄ (pH 7.0) containing 0.2 M NaCl; flow rate: 0.7 mL/min; temperature: 25 °C; detection: UV at 215 nm), to identify the composition variation of the ejection liquid.

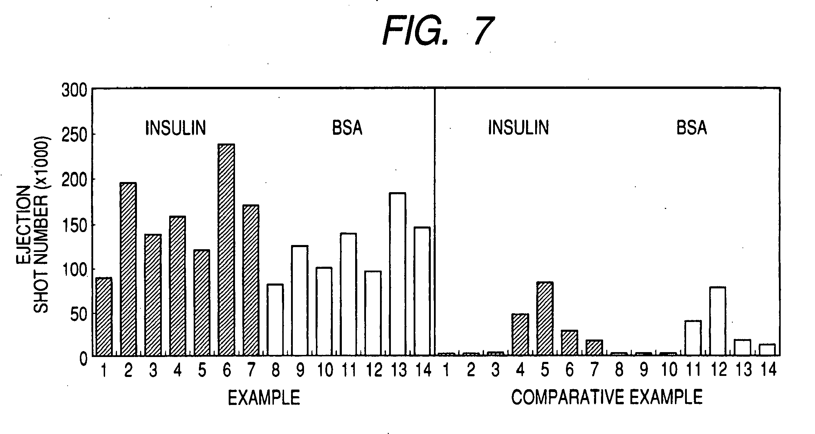

As Comparative Examples, insulin solutions and BSA solutions were prepared, and ejection liquids were prepared by adding to insulin or BSA a compound other than the compound having a guanidine group, and a carboxyl group or a sulfonic acid group in one molecule. These solutions and ejection liquids were subjected to the droplet ejection experiment in the same manner as in Examples. The formulations examined in Examples and Comparative Examples are listed in Table 1 shown below, and the results are shown in FIG. 7.

**Table 1**

| | Protein | Protein concentration (mg/mL) | Additive | Additive concentration (mg/mL) |
|---|---|---|---|---|
| Example 1 | Insulin | 4.0 | 2-Guanidinoacetic acid | 2.5 |
| Example 2 | Insulin | 4.0 | 3-Guanidinopropionic acid | 2.5 |
| Example 3 | Insulin | 4.0 | 4-Guanidinobutanoic acid | 2.5 |
| Example 4 | Insulin | 4.0 | α-Guanidinoglutamic acid | 2.5 |
| Example 5 | Insulin | 4.0 | Guanidinomethanesulfonic acid | 2.5 |
| Example 6 | Insulin | 4.0 | 2-Guanidinoethanesulfonic acid | 2.5 |
| Example 7 | Insulin | 4.0 | 3-Guanidinopropanesulfonic acid | 2.5 |
| Example 8 | BSA | 1.0 | 2-Guanidinoacetic acid | 2.5 |
| Example 9 | BSA | 1.0 | 3-Guanidinopropionic acid | 2.5 |
| Example 10 | BSA | 1.0 | 4-Guanidinobutanoic acid | 2.5 |
| Example 11 | BSA | 1.0 | α-Guanidinoglutamic acid | 2.5 |
| Example 12 | BSA | 1.0 | Guanidinomethanesulfonic acid | 2.5 |
| Example 13 | BSA | 1.0 | 2-Guanidinoethanesulfonic acid | 2.5 |
| Example 14 | BSA | 1.0 | 3-Guanidinopropanesulfonic acid | 2.5 |
| Comparative Example 1 | Insulin | 4.0 | None | - |
| Comparative Example 2 | Insulin | 4.0 | Sucrose | 2.5 |
| Comparative Example 3 | Insulin | 4.0 | Guanidine hydrochloride | 2.5 |
| Comparative Example 4 | Insulin | 4.0 | Arginine | 2.5 |
| Comparative Example 5 | Insulin | 4.0 | Arginine | 5.0 |
| Comparative Example 6 | Insulin | 4.0 | Tween 80 | 2.5 |
| Comparative Example 7 | Insulin | 4.0 | Tween 80 | 5.0 |
| Comparative Example 8 | BSA | 1.0 | None | - |
| Comparative Example 9 | BSA | 1.0 | Sucrose | 2.5 |
| Comparative Example 10 | BSA | 1.0 | Guanidine hydrochloride | 2.5 |
| Comparative Example 11 | BSA | 1.0 | Arginine | 2.5 |
| Comparative Example 12 | BSA | 1.0 | Arginine | 5.0 |
| Comparative Example 13 | BSA | 1.0 | Tween 80 | 2.5 |
| Comparative Example 14 | BSA | 1.0 | Tween 80 | 5.0 |

FIG. 7 shows that the solutions of Comparative Examples 1 to 3 and 8 to 10 were almost not ejected irrespective of the presence or absence of the additive. On the other hand, the additives such as arginine and Tween 80 enabled the ejection of the protein solutions. The increase of the addition amount of arginine increased the number of ejection shots, but contrarily the increase of the addition amount of Tween 80 decreased the number of ejection shots. On the contrary, in Examples 1 to 14, the protein solutions were able to be ejected with the numbers of ejection shots at least twice or more as large as those in the cases in Comparative Examples 4 and 11 where arginine was added. Additionally, because the numbers of ejection shots in Examples are the same as or more than those in Comparative Examples 5 and 12, conceivably the compounds in the present invention give the same effects as those due to arginine, with the concentrations half or less than the concentration of arginine. According to the results of the HPLC analysis, in any of Examples 1 to 14, neither the peak position variation nor the peak area variation, and no liquid composition variation were found between before and after the ejection.

### (Examples 15 to 21 and Comparative Example 15)

### (Ejection Frequency and Ejection Amount)

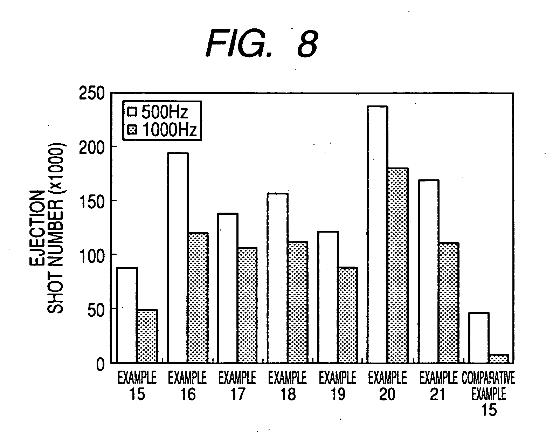

For the formulations of Examples 1 to 7 and Comparative Example 4, ejection was carried out under the same conditions as in Example 1, wherein the ejection frequency was varied from 500 Hz to 1000 Hz and the numbers of ejection shots were compared. The results thus obtained are shown in FIG. 8 wherein Examples 15 to 21 correspond to Examples 1 to 7, respectively, and Comparative Example 15 corresponds to Comparative Example 4.

By increasing the ejection frequency, the number of ejection shots was varied depending on the added compounds. A comparison of Examples with Comparative Example revealed that the difference in the number of ejection shots at 1000 Hz is 6 to 25 folds, verifying that the formulations of Examples were more effective for ejection at a high frequency.

### (Examples 22 to 61 and Comparative Examples 16 to 30)

### (Effects on Various Proteins and Concentrations of Additives)

Successively, as the compound having a guanidine group, and a carboxyl group or a sulfonic acid group in one molecule, 3-guanidinopropionic acid and 2-guanidinoethanesulfonic acid were selected and added to various proteins in certain concentrations. The ejection liquids thus obtained were evaluated by the ejection experiment. The ejection frequency was set at 1000 Hz. Each ejection experiment was carried out for 120 seconds, and a case where ejection was normal is marked with "A" and a case where ejection stopped halfway is marked with "C." The formulations examined and the results obtained in Examples and Comparative Examples are listed in Table 2 shown below.

**Table 2**

| | Protein | Protein concentration (mg/mL) | Additive | Additive concentration (mg/mL) | Ejection performance |
|---|---|---|---|---|---|
| Example 22 | Glucagon | 1.0 | 3-Guanidinopropionic acid | 2.5 | A |
| Example 23 | GLP-1 | 0.5 | 3-Guanidinopropionic acid | 1.0 | A |
| Example 24 | G-CSF | 1.0 | 3-Guanidinopropionic acid | 2.5 | A |
| Example 25 | IgE | 1.0 | 3-Guanidinopropionic acid | 2.5 | A |
| Example 26 | INF α | 1.0 | 3-Guanidinopropionic acid | 2.5 | A |
| Example 27 | INF γ | 1.0 | 3-Guanidinopropionic acid | 2.5 | A |
| Example 28 | Calcitonin | 1.0 | 3-Guanidinopropionic acid | 2.5 | A |
| Example 29 | IL-2 | 0.1 | 3-Guanidinopropionic acid | 0.5 | A |
| Example 30 | IL-6 | 0.1 | 3-Guanidinopropionic acid | 0.5 | A |
| Example 31 | TNF α | 1.0 | 3-Guanidinopropionic acid | 2.5 | A |
| Example 32 | Cytochrome c | 1.0 | 3-Guanidinopropionic acid | 2.5 | A |
| Example 33 | hGH | 2.0 | 3-Guanidinopropionic acid | 5.0 | A |
| Example 34 | EPO | 1.0 | 3-Guanidinopropionic acid | 2.5 | A |
| Example 35 | IL-6 receptor antibody | 0.1 | 3-Guanidinopropionic acid | 0.5 | A |
| Example 36 | VEGF antibody | 0.1 | 3-Guanidinopropionic acid | 0.5 | A |
| Example 37 | Insulin | 1.0 | 3-Guanidinopropionic acid | 1.0 | A |
| Example 38 | Insulin | 5.0 | 3-Guanidinopropionic acid | 2.5 | A |
| Example 39 | Insulin | 10 | 3-Guanidinopropionic acid | 50 | A |
| Example 40 | BSA | 1.0 | 3-Guanidinopropionic acid | 5.0 | A |
| Example 41 | BSA | 3.0 | 3-Guanidinopropionic acid | 50 | A |
| Example 42 | Glucagon | 1.0 | 2-Guanidinoethanesulfonic acid | 2.5 | A |
| Example 43 | GLP-1 | 0.5 | 2-Guanidinoethanesulfonic acid | 1.0 | A |
| Example 44 | G-CSF | 1.0 | 2-Guanidinoethanesulfonic acid | 2.5 | A |
| Example 45 | IgE | 1.0 | 2-Guanidinoethanesulfonic acid | 2.5 | A |
| Example 46 | INF α | 1.0 | 2-Guanidinoethanesulfonic acid | 2.5 | A |
| Example 47 | INF γ | 1.0 | 2-Guanidinoethanesulfonic acid | 2.5 | A |
| Example 48 | Calcitonin | 1.0 | 2-Guanidinoethanesulfonic acid | 2.5 | A |
| Example 49 | IL-2 | 0.1 | 2-Guanidinoethanesulfonic acid | 0.5 | A |
| Example 50 | IL-6 | 0.1 | 2-Guanidinoethanesulfonic acid | 0.5 | A |
| Example 51 | TNF α | 1.0 | 2-Guanidinoethanesulfonic acid | 2.5 | A |
| Example 52 | Cytochrome c | 1.0 | 2-Guanidinoethanesulfonic acid | 2.5 | A |
| Example 53 | hGH | 2.0 | 2-Guanidinoethanesulfonic acid | 5.0 | A |
| Example 54 | EPO | 1.0 | 2-Guanidinoethanesulfonic acid | 2.5 | A |
| Example 55 | IL-6 receptor antibody | 0.1 | 2-Guanidinoethanesulfonic acid | 0.5 | A |
| Example 56 | VEGF antibody | 0.1 | 2-Guanidinoethanesulfonic acid | 0.5 | A |
| Example 57 | Insulin | 1.0 | 2-Guanidinoethanesulfonic acid | 1.0 | A |
| Example 58 | Insulin | 5.0 | 2-Guanidinoethanesulfonic acid | 5.0 | A |
| Example 59 | Insulin | 10 | 2-Guanidinoethanesulfonic acid | 30 | A |
| Example 60 | BSA | 1.0 | 2-Guanidinoethanesulfonic acid | 4.0 | A |
| Example 61 | BSA | 3.0 | 2-Guanidinoethanesulfonic acid | 40 | A |
| Comparative Example 16 | Glucagon | 1.0 | None | - | C |
| Comparative Example 17 | GLP-1 | 0.5 | None | - | C |
| Comparative Example 18 | G-CSF | 1.0 | None | - | C |
| Comparative Example 19 | IgE | 1.0 | None | - | C |
| Comparative Example 20 | INF α | 1.0 | None | - | C |
| Comparative Example 21 | INF γ | 1.0 | None | - | C |
| Comparative Example 22 | Calcitonin | 1.0 | None | - | C |
| Comparative Example 23 | IL-2 | 0.1 | None | - | C |
| Comparative Example 24 | IL-6 | 0.1 | None | - | C |
| Comparative Example 25 | TNF α 1.0 | | None | - | C |
| Comparative Example 26 | Insulin | 1.0 | None | - | C |
| Comparative Example 27 | hGH | 2.0 | None | - | C |
| Comparative Example 28 | EPO | 1.0 | None | - | C |
| Comparative Example 29 | IL-6 receptor antibody | 0.1 | None | - | C |
| Comparative Example | VEGF 30 antibody | 0.1 | None | - | C |

By adding 3-guanidinopropionic acid or 2-guanidinoethanesulfonic acid, the ejection based on the thermal inkjet system was normally carried out for proteins and peptides. Consequently, 3-guanidinopropionic acid and 2-guanidinoethanesulfonic acid were verified to display effects on wide ranges of proteins and peptides. As the results of the HPLC analysis applied to Examples in which normal ejection was carried out, neither the peak chart variations nor the liquid composition variations between before and after ejection were found.

### (Examples 62 to 82)

### (Synergetic Effects due to the Compound Having a Guanidine Group, and a Carboxyl Group or a Sulfonic Acid Group in One Molecule and a Surfactant)

To the protein solutions and the peptide solutions, 3-guanidinopropionic acid was added, and further a surfactant was added to prepare ejection liquids. The ejection liquids were evaluated by the same ejection experiment as in Example 22. The formulations examined in present Examples and the results thus obtained are listed in Table 3 shown below.

**Table 3**

| | Protein | Protein concentration (mg/mL) | 3-Guanidino-propionic acid concentration (mg/mL) | Tween 80 concentration (mg/mL) | Ejection performance |
|---|---|---|---|---|---|
| Example 62 | Glucagon | 1.0 | 0.5 | 0.25 | A |
| Example 63 | GLP-1 | 0.5 | 0.1 | 0.1 | A |
| Example 64 | G-CSF | 1.0 | 0.5 | 0.25 | A |
| Example 65 | IgE | 1.0 | 0.5 | 0.25 | A |
| Example 66 | INF α | 1.0 | 0.5 | 0.1 | A |
| Example 67 | INF γ | 1.0 | 0.5 | 0.1 | A |
| Example 68 | Calcitonin | 1.0 | 0.5 | 0.1 | A |
| Example 69 | IL-2 | 0.1 | 0.1 | 0.1 | A |
| Example 70 | IL-6 | 0.1 | 0.1 | 0.1 | A |
| Example 71 | TNF α | 1.0 | 0.5 | 0.25 | A |
| Example 72 | Cytochrome c | 1.0 | 0.2 | 0.1 | A |
| Example 73 | hGH | 2.0 | 1.0 | 0.50 | A |
| Example 74 | EPO | 1.0 | 0.5 | 0.25 | A |
| Example 75 | IL-6 receptor antibody | 0.1 | 0.1 | 0.1 | A |
| Example 76 | VEGF antibody | 0.1 | 0.1 | 0.1 | A |
| Example 77 | Insulin | 5.0 | 1.0 | 0.5 | A |
| Example 78 | Insulin | 10 | 5.0 | 1.0 | A |
| Example 79 | Insulin | 40 | 50 | 5.0 | A |
| Example 80 | BSA | 1.0 | 0.5 | 0.5 | A |
| Example 81 | BSA | 5.0 | 5.0 | 1.0 | A |
| Example 82 | BSA | 10 | 100 | 10 | A |

When 3-guanidinopropionic acid and Tween (trade mark) 80 (polyoxyethylene 20 sorbitan monooleate: manufactured by Calbiochem Inc.) were added simultaneously, the protein and peptide solutions were able to be ejected with the concentrations of 3-guanidinopropionic acid reduced by a factor of 5 to 10 as compared to the cases where merely 3-guanidinopropionic acid was added. The total amounts of the additives can also be drastically reduced, and additionally, ejection was enabled even for those protein concentrations that had not allowed ejection merely with 3-guanidinopropionic acid; such synergetic effects also enabled to eject protein solutions with higher concentrations. Examples were subjected to the HPLC analysis and consequently no peak chart variations and no liquid composition variations were found between before and after ejection.

### (Examples 83 to 131 and Comparative Examples 31 to 35)

A liquid ejection head, of 3 µm in nozzle diameter, based on the thermal inkjet system was prepared, and a reservoir connected to the liquid ejection head was filled with a 30% aqueous solution of ethanol. By driving the ejection head with a controller electrically connected to the liquid ejection head, the liquid was ejected from the ejection nozzle, and the particle size and the particle size distribution of the obtained droplets were measured with a laser diffraction particle size distribution analyzer (manufactured by Spraytec, Malvern Instruments, Ltd.) and were identified. Consequently, the droplets were detected as droplets having a sharp particle size distribution at about 3 µm.

Each of the prepared ejection liquids was filled in the above-described head cartridge of 3 µm in nozzle diameter, the head cartridge was connected to the ejection controller, and the ejection liquid was ejected at a frequency of 20 kHz for 1 second, and after an interval of 60 seconds, the next ejection was carried out. This was repeated 50 times, and whether or not the liquid was ejected was checked by visual inspection. A case where the ejection was observed for 50 times was evaluated as "A", and a case where the ejection stopped halfway was evaluated as "C". The formulations examined and the results obtained in Examples and Comparative Examples are listed in Tables 4 to 6 shown below.

**Table 4**

| | Protein | Protein concentration (mg/mL) | Additive | Additive concentration (mg/mL) | Tween 80 concentration (mg/mL) | Ejection performance |
|---|---|---|---|---|---|---|
| Example 83 | Glucagon | 1.0 | 3-Guanidinopropionic acid | 25 | - | A |
| Example 84 | GLP-1 | 0.5 | 3-Guanidinopropionic acid | 15 | - | A |
| Example 85 | G-CSF | 1.0 | 3-Guanidinopropionic acid | 25 | - | A |
| Example 86 | IgE | 1.0 | 3-Guanidinopropionic acid | 25 | - | A |
| Example 87 | INF α | 1.0 | 3-Guanidinopropionic acid | 25 | - | A |
| Example 88 | INF γ | 1.0 | 3-Guanidinopropionic acid | 25 | - | A |
| Example 89 | Calcitonin | 1.0 | 3-Guanidinopropionic acid | 25 | - | A |
| Example 90 | IL-2 | 0.1 | 3-Guanidinopropionic acid | 2.0 | - | A |
| Example 91 | IL-6 | 0.1 | 3-Guanidinopropionic acid | 2.0 | - | A |
| Example 92 | TNF α | 1.0 | 3-Guanidinopropionic acid | 25 | - | A |
| Example 93 | hGH | 2.0 | 3-Guanidinopropionic acid | 50 | - | A |
| Example 94 | EPO | 1.0 | 3-Guanidinopropionic acid | 25 | - | A |
| Example 95 | IL-6 receptor antibody | 0.1 | 3-Guanidinopropionic acid | 2.0 | - | A |
| Example 96 | VEGF antibody | 0.1 | 3-Guanidinopropionic acid | 2.0 | - | A |
| Example 97 | Insulin | 1.0 | 3-Guanidinopropionic acid | 20 | - | A |
| Example 98 | Insulin | 5.0 | 3-Guanidinopropionic acid | 40 | - | A |
| Example 99 | Insulin | 10 | 3-Guanidinopropionic acid | 100 | - | A |

**Table 5**

| | Protein | Protein concentration (mg/mL) | Additive | Additive concentration (mg/mL) | Tween 80 concentration (mg/mL) | Ejection performance |
|---|---|---|---|---|---|---|
| Example 100 | Glucagon | 1.0 | 2-Guanidinoethanesulfonic acid | 20 | - | A |
| Example 101 | GLP-1 | 0.5 | 2-Guanidinoethanesulfonic acid | 10 | - | A |
| Example 102 | G-CSF | 1.0 | 2-Guanidinoethanesulfonic acid | 20 | - | A |
| Example 103 | IgE | 1.0 | 2-Guanidinoethanesulfonic acid | 20 | - | A |
| Example 104 | INF α | 1.0 | 2-Guanidinoethanesulfonic acid | 20 | - | A |
| Example 105 | INF γ | 1.0 | 2-Guanidinoethanesulfonic acid | 20 | - | A |
| Example 106 | Calcitonin | 1.0 | 2-Guanidinoethanesulfonic acid | 20 | - | A |
| Example 107 | IL-2 | 0.1 | 2-Guanidinoethanesulfonic acid | 2.0 | - | A |
| Example 108 | IL-6 | 0.1 | 2-Guanidinoethanesulfonic acid | 2.0 | - | A |
| Example 109 | TNF α | 1.0 | 2-Guanidinoethanesulfonic acid | 20 | - | A |
| Example 110 | hGH | 2.0 | 2-Guanidinoethanesulfonic acid | 40 | - | A |
| Example 111 | EPO | 1.0 | 2-Guanidinoethanesulfonic acid | 20 | - | A |
| Example 112 | IL-6 receptor antibody | 0.1 | 2-Guanidinoethanesulfonic acid | 2.0 | - | A |
| Example 113 | VEGF antibody | 0.1 | 2-Guanidinoethanesulfonic acid | 2.0 | - | A |
| Example 114 | Insulin | 1.0 | 2-Guanidinoethanesulfonic acid | 20 | - | A |
| Example 115 | Insulin | 5.0 | 2-Guanidinoethanesulfonic acid | 30 | - | A |
| Example 116 | Insulin | 10 | 2-Guanidinoethanesulfonic acid | 8.0 | - | A |
| Example 117 | Glucagon | 1.0 | 3-Guanidinopropionic acid | 1.0 | 0.25 | A |
| Example 118 | GLP-1 | 0.5 | 3-Guanidinopropionic acid | 0.5 | 0.25 | A |
| Example 119 | IL-6 | 0.1 | 3-Guanidinopropionic acid | 0.5 | 0.1 | A |
| Example 120 | TNF α | 1.0 | 3-Guanidinopropionic acid | 1.0 | 0.25 | A |
| Example 121 | hGH | 2.0 | 3-Guanidinopropionic acid | 2.0 | 0.5 | A |
| Example 122 | EPO | 1.0 | 3-Guanidinopropionic acid | 1.0 | 0.25 | A |
| Example 123 | IL-6 receptor antibody | 0.1 | 3-Guanidinopropionic acid | 0.5 | 0.1 | A |
| Example 124 | VEGF antibody | 0.1 | 3-Guanidinopropionic acid | 0.5 | 0.1 | A |
| Example 125 | Insulin | 1.0 | 3-Guanidinopropionic acid | 0.1 | 0.1 | A |
| Example 126 | Insulin | 5.0 | 3-Guanidinopropionic acid | 5.0 | 0.5 | A |
| Example 127 | Insulin | 10 | 3-Guanidinopropionic acid | 10 | 5.0 | A |
| Example 128 | Insulin | 40 | 3-Guanidinopropionic acid | 100 | 10 | A |
| Example 129 | BSA | 1.0 | 3-Guanidinopropionic acid | 1.0 | 1.0 | A |
| Example 130 | BSA | 5.0 | 3-Guanidinopropionic acid | 25 | 5.0 | A |
| Example 131 | BSA | 10 | 3-Guanidinopropionic acid | 150 | 10 | A |

**Table 6**

| | Protein | Protein concentration tion (mg/mL) | Additive | Additive concentration (mg/mL) | Tween 80 concentration (mg/mL) | Ejection performance |
|---|---|---|---|---|---|---|
| Comparative Example 31 | Insulin | 1.0 | - | - | - | C |
| Comparative Example 32 | Insulin | 1.0 | Arginine | 10 | - | C |
| Comparative Example 33 | Insulin | 1.0 | Arginine | 50 | - | A |
| Comparative Example 34 | Insulin | 1.0 | Arginine | 1.0 | 0.5 | C |
| Comparative Example 35 | Insulin | 1.0 | Arginine | 10 | 10 | A |

In the thermal inkjet of 3 µm in nozzle diameter, Comparative Example 31 was not ejected because Comparative Example 31 did not contain any additive. In Comparative Examples 32 to 35, it was verified that when the additive concentration was high, the ejection liquid were stably ejected. On the other hand, it was verified that in Examples, the ejection liquids were stably ejected with lower additive concentrations as compared to Comparative Examples, and it was verified that the addition effect of the compounds of the present invention is high. As the results of the HPLC analysis, in each of Examples, neither the peak position variation nor the peak area variation, and no liquid composition variation were found between before and after ejection.

### (Example 132)

### (Preparation of an Antibody Chip by Using an Inkjet

### Printer and Sensing)

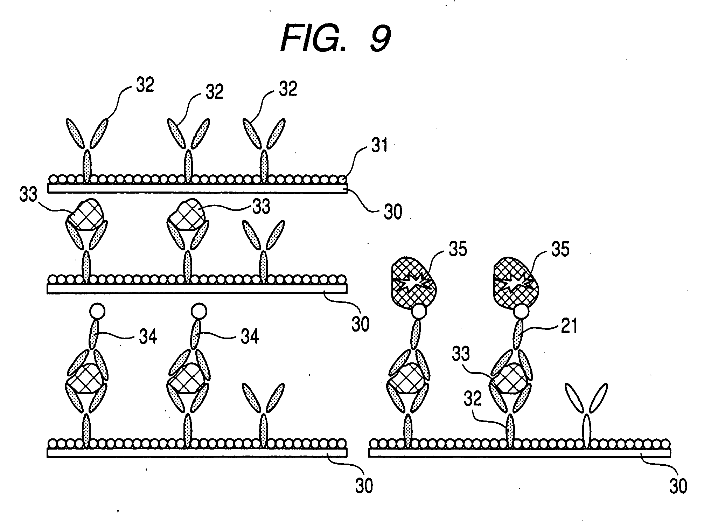

FIG. 9 shows a model view of the present Example, where are shown: a substrate 30; a masking agent 31; a substance 32 representing a substance specifically reacting with the substance to be tested, a protein, a peptide or the like; a substance 33 to be tested; a substance 34 specific to the substance to be tested; and a label 35. The solutions of Human IL2 monoclonal antibody, Human IL4 monoclonal antibody and Human IL6 monoclonal antibody were prepared by using PBS each in a concentration of 0.1 to 500 µg/mL. To these solutions 2-guanidinoethanesulfonic acid was added in a content of 0.5% (w/w), and thus ejection liquids were prepared. These liquids were each filled in the head of an inkjet printer (trade name: PIXUS950i; manufactured by Canon Inc.) and ejected onto a poly-L-lysin coated slide glass.

The glasses after ejection were incubated at 4 °C, and the glasses after incubation were subjected to masking with 1% BSA. After masking, the glasses were washed thoroughly, and thus antibody chip substrates were prepared.

Then, the ejection liquids of the recombinants IL2, IL4 and IL6, as the substances 33 to be tested corresponding to the chips, were prepared each in a concentration of 1 µg/mL so as to concomitantly contain 0.5% of 2-guanidinoethanesulfonic acid (w/w), 0.5% of Tween 20 (w/w) and 0.1% of BSA (w/w) by using PBS. These liquids were each filled in the head of an inkjet printer (trade name: PIXUS950i; manufactured by Canon Inc.) and ejected in the same pattern onto the above-described substrates. After ejection, the substrates were each covered with a cover glass and were allowed to react at 4°C. After reaction, the substrates were thoroughly washed and dried.

Next, the substances 34 to form specific bonds with the samples were reacted with the substrates, and thereafter, the substances were applied with labels 35. As the substances to form specific bonds with the samples, the 1 µg/mL antibody solutions of the respective biotin-labeled antibodies (biotinized Human IL2 monoclonal antibody, biotinized Human IL4 monoclonal antibody and biotinized Human IL6 monoclonal antibody) were prepared with PBS so as to each have the following final concentrations: 0.5% of 2-guanidinoethanesulfonic acid (w/w), 0.5% of Tween 20 (w/w) and 0.1% of BSA (w/w). These ejection liquids were each filled in the head of an inkjet printer (trade name: PIXUS950i; manufactured by Canon Inc.) and ejected in the same pattern onto the above-described substrates. After ejection, the substrates were each covered with a cover glass and were allowed to react at 4°C. After reaction, the substrates were thoroughly washed and dried.

For labeling, a 10 µg/mL solution of Cy3-labeled streptavidin was prepared with PBS so as to have the following final concentrations: 0.5% of 2-guanidinoethanesulfonic acid (w/w), 0.5% of Tween 20 (w/w) and 0.1% of BSA (w/w). The ejection liquid was filled in the head of an inkjet printer (trade name: PIXUS950i; manufactured by Canon Inc.) and ejected in the same pattern onto the above-described substrates. After ejection, the substrates were each covered with a cover glass and were allowed to react at 4°C. After reaction, the substrates were thoroughly washed and dried.

Subsequently, each of the substrates after reaction was irradiated with excitation light, and the emitted light quantity of Cy3 was measured as the fluorescence signal quantity by using a fluorescence scanner equipped with a filter having a transmissive wavelength of 532 nm. Consequently, the fluorescence signals corresponding to the types and concentrations of the samples were able to be detected.

The present invention is not limited to the above embodiments and various changes and modifications can be made within the spirit and scope of the present invention. Therefore to apprise the public of the scope of the present invention, the following claims are made.

This application claims priority from Japanese Patent Application No. 2006-327031 filed on December 4, 2006.

## Claims

1. An ejection liquid for ejection by imparting thermal energy, comprising:
a protein or a peptide;
a compound having a guanidine group, and a carboxyl group or a sulfonic acid group in one molecule, and being represented by the general formula (1), or a salt of the compound; and
a liquid medium: 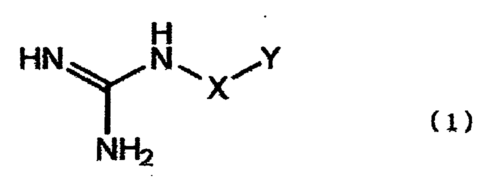
wherein X in the general formula (1) represents an optionally branched alkyl group having one or more and eight or less carbon atoms, wherein when a side chain branched at the alkyl group X of formula (1) has a functional group or functional groups, the side chain has one or more hydroxyl groups, carboxyl groups or sulfonic acid groups, and Y represents a carboxyl group or a sulfonic acid group.

2. The ejection liquid according to claim 1, wherein the compound represented by the general formula (1) comprises one or two or more compounds selected from the group consisting of 2-guanidinoacetic acid, 3-guanidinopropionic acid, 4-guanidinobutanoic acid, α-guanidinoglutamic acid, guanidinomethanesulfonic acid, 2-guanidinoethanesulfonic acid, 3-guanidinopropanesulfonic acid, and the salts of these

3. The ejection liquid according to claim 1 or 2, further comprising a surfactant.

4. The ejection liquid according to claim 3, wherein the surfactant is a polyoxyethylenesorbitan fatty acid ester.

5. A liquid ejection cartridge comprising:
a reservoir in which the ejection liquid according to any one of claims 1 to 4 is stored; and
an ejection head comprising an electrothermal transducer to impart thermal energy to the ejection liquid.

6. An inhaler comprising:
the cartridge according to claim 5; and
a suction port from which a user inhales the liquid ejected from the ejection head of the cartridge .

7. An ejection method for ejecting the liquid composition, by imparting thermal energy thereto, comprising:
a protein or a peptide;
a compound having a guanidine group, and a carboxyl group or a sulfonic acid group in one molecule, and being represented by the general formula (1), or a salt of the compound; and
a liquid medium: 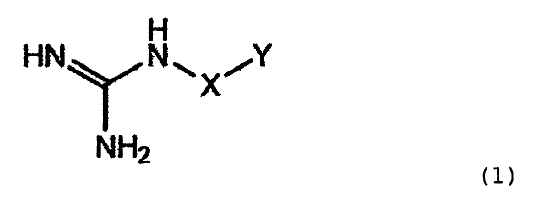
wherein X in the general formula (1) represents an optionally branched alkyl group having one or more and eight or less carbon atoms, wherein when a side chain branched at the alkyl group X of formula (1) has a functional group or functional groups, the side chain has one or more hydroxyl groups, carboxyl groups or sulfonic acid groups, and Y represents a carboxyl group or a sulfonic acid group.

8. The ejection method according to claim 7, wherein the liquid composition further comprises a surfactant.

9. The ejection method according to claim 7 or 8, wherein the method for ejecting the liquid composition by imparting thermal energy thereto is based on a thermal inkjet system.

10. A use of a compound having a guanidine group, and a carboxyl group or a sulfonic acid group in one molecule, and being represented by the following general formula, or a salt of the compound, as an additive for ejecting a liquid composition comprising a protein and a peptide by imparting thermal energy to the liquid composition: 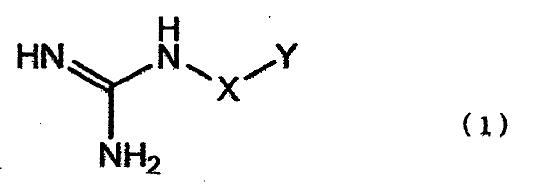 wherein X in the general formula (1) represents an optionally branched alkyl group having one or more and eight or less carbon atoms, wherein when a side chain branched at the alkyl group X of formula (1) has a functional group or functional groups, the side chain has one or more hydroxyl groups, carboxyl groups or sulfonic acid groups, and Y represents a carboxyl group or a sulfonic acid group.

## Patentansprüche

1. Ausstoßflüssigkeit zum Ausstoßen durch Vermitteln von thermischer Energie, umfassend:
ein Protein oder ein Peptid;
eine Verbindung die eine Guanidingruppe, und eine Carboxylgruppe oder eine Sulfonsäuregruppe in einem Molekül aufweist, und die durch die allgemeine Formel (1) dargestellt ist oder ein Salz der Verbindung; und
ein flüssiges Medium: 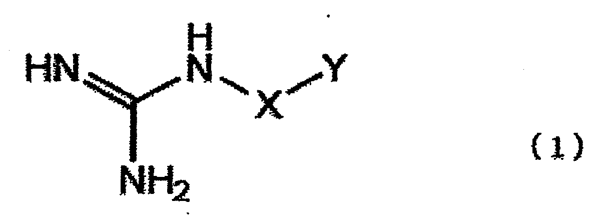
wobei X in der allgemeinen Formel (1) eine optional verzweigte Alkylgruppe darstellt, die ein oder mehr und acht oder weniger Kohlenstoffatome aufweist, wobei, falls eine an der Alkylgruppe X der Formel (1) verzweigte Seitenkette eine funktionelle Gruppe oder funktionelle Gruppen aufweist, die Seitenkette eine oder mehr Hydroxylgruppen, Carboxylgruppen oder Sulfonsäuregruppen aufweist, und Y eine Carboxylgruppe oder eine Sulfonsäuregruppe darstellt.

2. Ausstoßflüssigkeit nach Anspruch 1, wobei die durch die allgemeine Formel (1) dargestellte Verbindung, eine oder zwei oder mehr Verbindungen ausgewählt aus der Gruppe, bestehend aus 2-Guanidinessigsäure, 3-Guanidinpropionsäure, 4-Guanidinbutansäure, α-Guanidinglutaminsäure, Guanidinmethansulfonsäure, 2-Guanidinethansulfonsäure, 3-Guanidinpropansulfonsäure, und Salze davon umfasst.

3. Ausstoßflüssigkeit nach dem Anspruch 1 oder 2, die ferner ein Tensid umfasst.

4. Ausstoßflüssigkeit nach dem Anspruch 3, wobei das Tensid ein Polyoxyethylensorbitanfettsäureester ist.

5. Flüssigkeitsausstoßkartusche, umfassend:
ein Reservoir, in welchem die Ausstoßflüssigkeit nach einem der Ansprüche 1 bis 4 gespeichert ist; und
ein Ausstoßkopf, welcher einen elektrothermischen Wandler umfasst, um thermische Energie an die Ausstoßflüssigkeit zu vermitteln.

6. Inhalator, umfassend:
die Kartusche nach Anspruch 5, und
einen Sauganschluss, von welchem ein Benutzer die vom Ausstoßkopf der Kartusche ausgestoßene Flüssigkeit inhaliert.

7. Ausstoßverfahren zum Ausstoßen der Flüssigkeitszusammensetzung durch Vermitteln von thermischer Energie an diese, umfassend:
ein Protein oder ein Peptid,
eine Verbindung die eine Guanidingruppe, und eine Carboxylgruppe oder eine Sulfonsäuregruppe in einem Molekül aufweist, und die durch die allgemeine Formel (1) dargestellt ist oder ein Salz der Verbindung; und
ein flüssiges Medium: 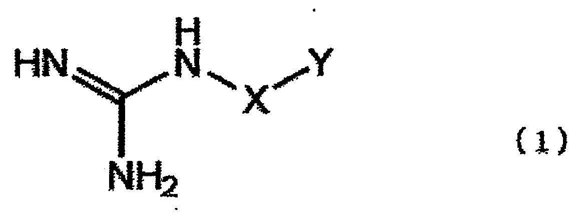
wobei X in der allgemeinen Formel (1) eine optional verzweigte Alkylgruppe darstellt, die ein oder mehr und acht oder weniger Kohlenstoffatome aufweist, wobei, falls eine an der Alkylgruppe X der Formel (1) verzweigte Seitenkette eine funktionelle Gruppe oder funktionelle Gruppen aufweist, die Seitenkette eine oder mehr Hydroxylgruppen, Carboxylgruppen oder Sulfonsäuregruppen aufweist, und Y eine Carboxylgruppe oder eine Sulfonsäuregruppe darstellt.

8. Ausstoßverfahren nach Anspruch 7, wobei die Flüssigkeitszusammensetzung ferner ein Tensid umfasst.

9. Ausstoßverfahren nach Anspruch 7 oder 8, wobei das Verfahren zum Ausstoßen der Flüssigkeitszusammensetzung durch Vermitteln von thermischer Energie an diese auf einem thermischen Tintenstrahlsystem basiert.

10. Verwendung einer Verbindung, die eine Guanidingruppe, und eine Carboxylgruppe oder eine Sulfonsäuregruppe in einem Molekül aufweist, und die durch die folgende allgemeine Formel dargestellt ist, oder ein Salz der Verbindung, als ein Additiv, zum Ausstoßen einer Flüssigkeitszusammensetzung, die ein Protein und ein Peptid, durch Vermitteln von thermischer Energie zu der Flüssigkeitszusammensetzung, umfasst: 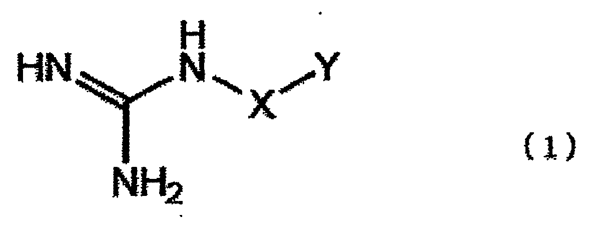 wobei X in der allgemeinen Formel (1) eine optional verzweigte Alkylgruppe darstellt, die ein oder mehr und acht oder weniger Kohlenstoffatome aufweist, wobei, falls eine an der Alkylgruppe X der Formel (1) verzweigte Seitenkette eine funktionelle Gruppe oder funktionelle Gruppen aufweist, die Seitenkette eine oder mehr Hydroxylgruppen, Carboxylgruppen oder Sulfonsäuregruppen aufweist, und Y eine Carboxylgruppe oder eine Sulfonsäuregruppe darstellt.

## Revendications

1. Liquide éjectable destiné à être éjecté par application d'énergie thermique, comprenant:
une protéine ou un peptide ;
un composé ayant un groupe guanidine et un groupe carboxyle ou un groupe acide sulfonique dans une molécule, et étant représenté par la formule générale (1), ou un sel du composé ; et
un milieu liquide : 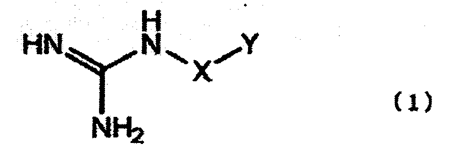
où X dans la formule générale (1) représente un groupe alkyle, éventuellement ramifié, ayant un à huit atomes de carbone, où, lorsqu'une chaîne latérale de ramification au niveau du groupe alkyle X de la formule (1) possède un ou plusieurs groupes fonctionnels, la chaîne latérale possède un ou plusieurs groupes hydroxyle, groupes carboxyle ou groupes acide sulfonique, et Y représente un groupe carboxyle ou un groupe acide sulfonique.

2. Liquide éjectable suivant la revendication 1, dans lequel le composé représenté par la formule générale (1) comprend un, deux ou plus de deux composés choisis dans le groupe consistant en l'acide 2-guanidinoacétique, l'acide 3-guanidinopropionique, l'acide 4-guanidinobutanoïque, l'acide α-guanidinoglutamique, l'acide guanidinométhane-sulfonique, l'acide 2-guanidinoéthanesulfonique, l'acide 3-guanidinopropanesulfonique et les sels de ces acides.

3. Liquide éjectable suivant la revendication 1 ou 2, comprenant en outre un agent tensioactif.

4. Liquide éjectable suivant la revendication 3, dans lequel l'agent tensioactif est un ester d'acide gras de polyoxyéthylène-sorbitanne.

5. Cartouche d'éjection de liquide, comprenant :
un réservoir dans lequel le liquide éjectable suivant l'une quelconque des revendications 1 à 4 est stocké ; et
une tête d'éjection comprenant un transducteur électrothermique pour l'application d'énergie thermique au liquide éjectable.

6. Inhalateur comprenant :
la cartouche suivant la revendication 5 ; et
un orifice d'aspiration par lequel un utilisateur inhale le liquide éjecté par la tête d'éjection de la cartouche.

7. Méthode d'éjection pour éjecter la composition liquide en appliquant de l'énergie thermique à celle-ci, comprenant :
une protéine ou un peptide ;
un composé ayant un groupe guanidine et un groupe carboxyle ou un groupe acide sulfonique dans une molécule, et étant représenté par la formule générale (1), ou un sel du composé ;
un milieu liquide : 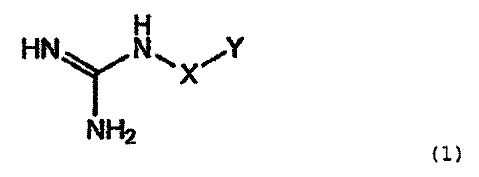
où X dans la formule générale (1) représente un groupe alkyle, éventuellement ramifié, ayant un à huit atomes de carbone, où, lorsqu'une chaîne latérale de ramification au niveau du groupe alkyle X de la formule (1) possède un ou plusieurs groupes fonctionnels, la chaîne latérale possède un ou plusieurs groupes hydroxyle, groupes carboxyle ou groupes acide sulfonique, et Y représente un groupe carboxyle ou un groupe acide sulfonique.

8. Méthode d'éjection suivant la revendication 7, dans laquelle la composition liquide comprend en outre un agent tensioactif.

9. Méthode d'éjection suivant la revendication 7 ou 8, dans laquelle la méthode d'éjection de la composition liquide par application d'énergie thermique à celle-ci est basée sur un système thermique à jet d'encre.

10. Utilisation d'un composé ayant un groupe guanidine et un groupe carboxyle ou un groupe acide sulfonique dans une molécule, et étant représenté par la formule générale suivante, ou d'un sel du composé, comme additif pour l'éjection d'une composition liquide comprenant une protéine et un peptide par application d'énergie thermique à la composition liquide : 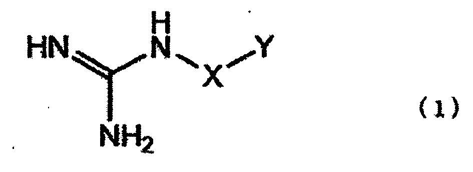 où X dans la formule générale (1) représente un groupe alkyle, éventuellement ramifié, ayant un à huit atomes de carbone, où, lorsqu'une chaîne latérale de ramification au niveau du groupe alkyle X de la formule (1) possède un ou plusieurs groupes fonctionnels, la chaîne latérale possède un ou plusieurs groupes hydroxyle, groupes carboxyle ou groupes acide sulfonique, et Y représente un groupe carboxyle ou un groupe acide sulfonique.
